# EUROPÄISCHE PATENTSCHRIFT

(11) **EP 1 267 845 B1**
(45) Veröffentlichungstag und Bekanntmachung des Hinweises auf die Patenterteilung: **03.01.2007**
(21) Anmeldenummer: 01919140.2
(22) Anmeldetag: 23.02.2001
(51) Int. Cl.: A61K 9/70

(54) **WIRKSTOFFPFLASTER**
PLASTER CONTAINING AN ACTIVE AGENT
EMPLATRE A PRINCIPE ACTIF

(30) Priorität: 20.03.2000 DE 10013504
(43) Veröffentlichungstag der Anmeldung: 02.01.2003
(73) Patentinhaber: Phoenix AG, 21079 Hamburg (DE)
(72) Erfinder: SCHUNK, Werner, 99867 Gotha (DE); BRUDER, Michael, 22147 Hamburg (DE); GIESSMANN, Konrad, 99867 Gotha (DE); KRAUSE, Karl-Heinz, 09123 Chemnitz (DE); MERKMANN, Gerhard, 99867 Gotha (DE)
(74) Vertreter: Finger, Karsten
(86) Internationale Anmeldenummer: PCT/DE2001/000687
(87) Internationale Veröffentlichungsnummer: WO 2001/070202

(56) Entgegenhaltungen:
- DD-A- 278 492
- DD-A- 278 494

## Beschreibung

Die Patentschriften DD 278492 A1 und DD 278494 A1 betreffen ein Wirkstoffpflaster, umfassend wenigstens
- eine Deckmembran und
- eine Wirkmembran auf der Basis einer hautverträglichen Matrix, in die ein Molekularsieb eingemischt ist, das mit wenigstens einem Wirkstoff beladen ist,
wobei das Molekularsieb/Wirkstoff-Addukt bei Hautkontakt der Wirkmembran in Verbindung mit dem Transpirationswasser der Haut den Wirkstoff freigibt.

Dabei ist die Wirkmembran insbesondere aus einem hautverträglichen Haftklebstoff gebildet, in die das Molekularsieb/Wirkstoff-Addukt eingemischt ist. Aus diesem Komplex wird der Wirkstoff desorptiv freigesetzt, indem Transpirationswasser der Haut in den Haftklebstoff eindiffundiert. Nachfolgend wird der Wirkstoff gelöst und diffundiert in Abhängigkeit von der Zusammensetzung des Haftklebstoffes an die Oberfläche der Haut.

Mit der Verwendung von Wirkstoffpflastern im Rahmen der medizinischen Behandlung sind folgende Vorteile verbunden:
- Wirkstoffpflaster bieten eine effektive Alternative zu Salbenverbänden.
- Wirkstoffpflaster erweitem das Therapiespektrum in der Medizin erheblich, insbesondere in der Dermatologie.
- Die Dauerapplikation von Wirkstoffpflastern ist häufig günstiger als bei Injektionen oder Tabletten. Sie beinhalten somit eine moderne Art der "Transdermalen Systeme".
- Da die Wirkmembran haftfreundlich ist, werden die äußeren Reize von Heftpflastern beseitigt. Trotzdem ist ein fester Sitz gewährleistet, womit eine gute Applikation verbunden ist. Diese Vorteile machen sich insbesondere bei der Behandlung von Kindern bemerkbar.

Im Rahmen einer Weiterentwicklung besteht die Aufgabe der Erfindung darin, ein Wirkstoffpflaster bereit zu stellen, das zusätzlich folgende Kriterien erfüllt:
- Eine hohe Wirkstoffkonzentration unmittelbar am Krankheitsherd mit kontinuierlichem Wirkstoffangebot muss gewährleistet sein. Bei den bisher bekannten Wirkstoffpflastern trat entweder ein "Spritzeneffekt" ein, d.h. ein schneller Abbau des Wirkstoffes und somit eine wesentliche Verringerung der medikamentösen Depotwirkung, oder die Freisetzung des Wirkstoffes verlief unkontrolliert.
- Das Wirkstoffpflaster muss sich ferner durch ein verbessertes Penetrationsverhalten im feuchten Milieu auszeichnen. Dies ist insofern wichtig, da das feuchte Milieu ideale Bedingungen für Wachstumsfaktoren der Zellen schafft und zur Immunabwehr dient.
- Darüber hinaus sollen mit dem Wirkstoffpflaster bei geringerer Medikamentenbelastung kürzere Behandlungszeiten erreicht werden, verbunden mit einer vereinfachten Überwachung ohne Klinikeinweisung.
- Schließlich soll mit dem Wirkstoffpflaster das medizinische Anwendungsspektrum erweitert werden, beispielsweise in der Dermatologie, zumal einige Hauterkrankungen (Granuloman, Verhornungen) nur mit Wirkstoffpflastern zu behandeln sind.

Zwecks Lösung dieser Aufgabe zeichnet sich nun das erfindungsgemäße Wirkstoffpflaster nach dem Kennzeichen des Patentanspruches 1 dadurch aus, dass
- das Molekularsieb/Wirkstoff-Addukt zusätzlich Kristallwasser enthält, und zwar derart, dass in Bezug auf eine ausreichende Grundmolmenge (m) an Kristallwasser
- das Molekularsieb partiell dehydratisiert ist, wobei das Molekularsieb mit der reduzierten Molmenge (m') an Kristallwasser mit dem Wirkstoff beladen ist, so dass bei Hautkontakt der Wirkmembran eine Adsorption von Wasser unter Desorption des Wirkstoffes stattfindet, wobei der Kristallwassergehalt des Molekularsiebes zunimmt.

Zweckmäßige Gestaltungsvarianten des Wirkstoffpflasters sind in den Patentansprüchen 2 bis 16 genannt.

Darüber hinaus besteht die Aufgabe der Erfindung darin, ein Verfahren zur Herstellung des erfindungsgemäßen Wirkstoffpflasters bereit zu stellen.

Gemäß Kennzeichen des Patentanspruches 17 zeichnet sich nun das Verfahren zur Herstellung des neuen Wirkstoffpflasters durch folgende Verfahrensschritte aus:
- das Molekularsieb mit einer ausreichenden Grundmolmenge (m) an Kristallwasser wird bei 300 bis 500°C, vorzugsweise bei 400 bis 450°C, mehrere Stunden, vorzugsweise 2 bis 6 Stunden, lang partiell dehydratisiert;
- anschließend wird das partiell dehydratisierte Molekularsieb mit der reduzierten Molmenge (m') an Kristallwasser mit wenigstens einem Wirkstoff unter Bildung des Molekularsieb/Wirkstoff-Adduktes beladen;
- nun wird das Molekularsieb/Wirkstoff-Addukt in die Matrix unter Bildung der Wirkmembran eingemischt;
- schließlich erfolgt die Konfektionierung der Wirkmembran mit der Deckmembran unter Bildung des Wirkstoffpflasters.

Die Dehydratisierung und/oder Beladung wird/werden dabei insbesondere in Gegenwart eines inerten Gases, beispielsweise von Stickstoff, durchgeführt. Die Dehydratisierung und/oder Beladung erfolgt/erfolgen ferner vorzugsweise unter Normaldruck. Die Beladung selbst kann beispielsweise mittels einer Kugelmühle vorgenommen werden.

Vorteilhafterweise schließt sich nach der Konfektionierung ein Trockenverfahren an, das bei 30 bis 100°C, vorzugsweise bei 50 bis 70°C, 2 bis 6 Stunden, vorzugsweise 3 bis 4 Stunden, lang durchgeführt wird. Auf diese Weise wird eine selbstklebende Matrix gebildet.

Alternativ hierzu kann das Molekularsieb/Wirkstoff-Addukt so präpariert sein, dass es in Verbindung mit einer nichtklebenden Matrix die Selbstklebefähigkeit der Wirkmembran auslöst.

Die Erfindung wird nun anhand von Ausführungsbeispielen unter Bezugnahme auf schematische Zeichnungen erläutert. Es zeigen:
- Fig. 1: den Adsorption/Desorptions-Mechanismus anhand von zwei Ausführungsbeispielen;
- Fig. 2: den Adsorption/Desorptions-Mechanismus anhand zweier Molekularsieb/Wirkstoff-Addukte, die einen unterschiedlichen Dehydratisierungsgrad aufweisen.

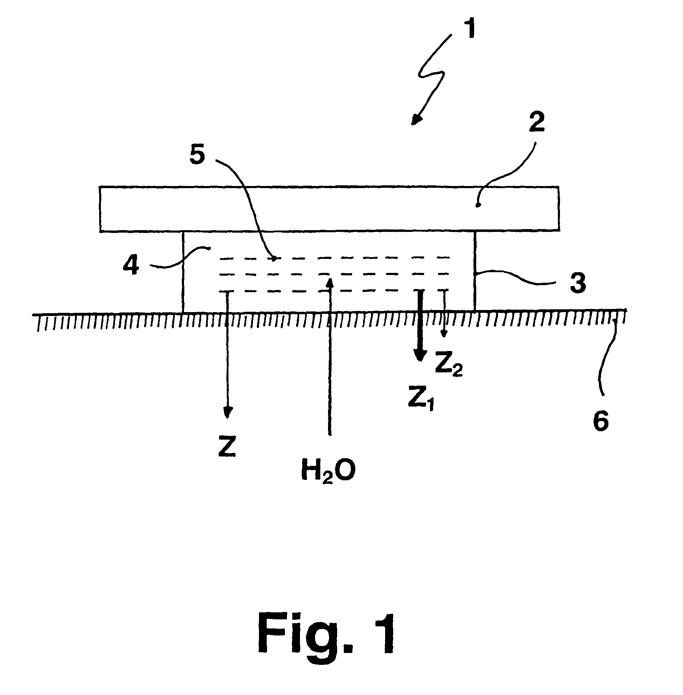

In Form eines Verbundkörpers umfasst das Wirkstoffpflaster 1 gemäß Fig. 1 eine Deckmembran 2 und eine selbstklebende Wirkmembran 3.

Die Wirkmembran 3 wiederum besteht aus der Matrix 4, in die das Molekularsieb/Wirkstoff-Addukt 5 eingemischt ist. Als Molekularsieb wird insbesondere ein Natrium-Aluminium-Silikat der Formel

Na₈₆ [(AlO₂)₈₆ · (SiO₂)₁₀₆] · m(m')H₂O

verwendet, das im noch nicht dehydratisierten Zustand eine Grundmolmenge (m = 276) an Kristallwasser enthält. Im Rahmen der partiellen Dehydratisierung werden dabei mindestens 20 %, vorzugsweise 40 bis 70 %, Mole Wasser entzogen. Das partiell dehydratisierte Molekularsieb mit der reduzierten Molmenge (m'; z.B. m' = 200) ist nun mit einem Wirkstoff Z beladen.

Bei Hautkontakt **6** der Wirkmembran **3** wird nun der Haut oder dem Wundbereich Wasser entzogen, das von dem Molekularsieb/Wirkstoff-Addukt **5** aufgenommen wird (Adsorption), wobei eine Desorption des Wirkstoffes **Z** stattfindet. Dabei nimmt der Kristallwassergehalt des Molekularsiebes zu. Das Molekularsieb erhält also das Kristallwasser zurück, das man ihm im Rahmen der partiellen Dehydratisierung entzogen hat.

Die Tatsache, dass das Motekularsieb/Wirkstoff-Addukt **5** grundsätzlich Kristallwasser enthält, sorgt für ein entsprechendes Feuchtmilieu. Auf diese Weise wird die Desorption des Wirkstoffes **Z** mittels des Lösungs- bzw. Dispergierungsmittels Wasser erleichtert. Zusätzlich schafft das feuchte Milieu ideale Bedingungen für Wachstumsfaktoren der Zellen und dient zudem zur lmmunabwehr.

Beispielhafte Wirkstoffpflaster sind:
Prednisolon-Wirkstoffpflaster (gegen Entzündungen)
Vitamin-A-Säure-Wirkstoffpflaster (gegen Verhornungen)
Antibiotikum-Wirkstoffpflaster (gegen Infektionskrankheiten)
Antithrombotikum-Wirkstoffplaster (gegen Thrombosen)

Im Rahmen der gleichen Figur ist ein weiteres Ausführungsbeispiel dargestellt. Hiernach ist das Molekularsieb bei einem einheitlichen Dehydratisierungsgrad mit einem ersten Wirkstoff **Z₁** und einem zweiten Wirkstoff **Z₂** beladen, die sich durch einen unterschiedlichen Beladungsgrad auszeichnen, so dass das Molekularsieb/Wirkstoff-Addukt **5** bei Hautkontakt **6** der Wirkmembran **3** die Wirkstoffe **Z₁** und **Z₂** sequentiell freigibt, was durch die unterschiedlichen Pfeilstärken dargestellt ist. Die beiden Wirkstoffe haben dabei etwa das gleiche Molekulargewicht. Folgendes Zahlenbeispiel soll dies verdeutlichen. Das Molekularsieb weist einen Dehydratisierungsgrad von 70 % auf. Bei einem Gesamtbeladungsgrad von 60 % an den Wirkstoffen **Z₁** und **Z₂** entfallen auf **Z₁** 40 % und **Z₂** 20 %. Aufgrund der größeren Beladungsmenge wird der Wirkstoff **Z₁** schneller abgegeben als der Wirkstoff **Z₂,** verbunden mit einer zeitlich versetzten Therapiewirkung.

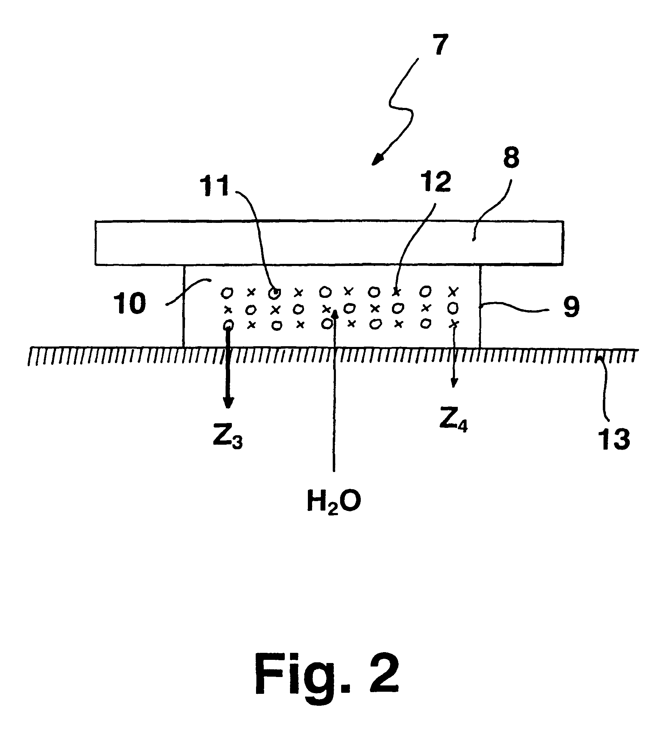

Eine beispielhafte medizinische Anwendung in diesem Sinne wäre die Kombination eines Antithrombotikums **(Z₁)** mit einem Antibiotikum **(Z₂),** das auch für das Ausführungsbeispiel gemäß Fig. 2 herangezogen werden kann, das nun näher vorgestellt wird.

In die Matrix **10** sind ein erstes und zweites Molekularsieb **11** bzw. **12** eingemischt, die typengleich sind, sich jedoch durch einen unterschiedlichen Dehydratisierungsgrad auszeichnen, wobei folgendes Beispiel genannt sei:

Molekularsiebtyp: Na₈₆ [(AlO₂)₈₆ · (SiO₂)₁₀₆] · m(m')H₂O
Grundmolmenge (m) vor der partiellen Dehydratisierung: m = 276
reduzierte Molmenge (m')
- Molekularsieb/Wirkstoff-Addukt **11** mit dem Wirkstoff **Z₃ :** m' = 100
- Molekularsieb/Wirkstoff-Addukt **12** mit dem Wirkstoff **Z₄ :** m' = 200

Bei etwa gleichem Molekulargewicht der Wirkstoffe **Z₃** und **Z₄** ist dabei jeweils der Beladungsgrad etwa gleich.

Bei Hautkontakt **13** der Wirkmembran **9** werden die Wirkstoffe **Z₃** und **Z₄** sequentiell freigegeben, was wiederum durch die unterschiedlichen Pfeilstärken symbolisiert ist. Mit anderen Worten: Das Addukt **11** mit dem größeren Dehydratisierungsgrad wird begieriger Wasser aufnehmen als das Addukt **12.** Folge ist, dass der Wirkstoff **Z₃** schneller abgegeben wird als der Wirkstoff **Z₄,** verbunden wiederum mit einer zeitlich versetzten Therapiewirkung.

Unabhängig von den Ausführungsbeispielen gelten für das Wirkstoffpflaster **1** und **7** zweckmäßigerweise folgende Parameter:
- Die Addukte **5, 11** und **12** sind innerhalb der Matrix **4** und **10** im wesentlichen gleichmäßig verteilt.
- Die Addukte **5, 11** und **12** weisen einen Anteil von 2 bis 20 Gew.-%, vorzugsweise 5 bis 10 Gew.-%, auf, und zwar bezogen auf die Gesamtmasse der Wirkmembran **3** und **9.**
- Die Matrix **4** und **10** ist ein Polymerwerkstoff, insbesondere wiederum ein Elastomer, ein thermoplastisches Elastomer oder ein thermoplastischer Kunststoff. Wichtig in diesem Zusammenhang ist, dass diese Werkstoffe eine hautverträgliche Matrix bilden.
- Die Wirkmembran **3** und **9** weist eine Schichtdicke von 0,5 mm bis 2 mm, vorzugsweise von 1 mm, auf. Dabei ist die Schichtdicke der Wirkmembran größer als die der Deckmembran.

Das Wirkstoffpflaster wird üblicherweise steril verpackt. Alternativ hierzu kann die Wirkmembran **3** und **9** mit einer abziehbaren Schutzfolie abgedeckt sein.

Die in der Beschreibung und in den Patentansprüchen erwähnten Aussagen zum Dehydratisierungsgrad und Beladungsgrad beziehen sich auf den Zustand nach Abschluss der partiellen Dehydratisierung bzw. der Beladung, d.h. ohne Stoffwechselaustausch von Wasser und Wirkstoff.

### Bezugszeichenliste

- 1.: Wirkstoffpflaster
- 2: Deckmembran
- 3: Wirkmembran
- 4: Matrix
- 5: Molekularsieb/Wirkstoff-Addukt mit dem Wirkstoff Z Molekularsieb/Wirkstoff-Addukt mit den Wirkstoffen Z₁, Z₂
- 6: Hautkontakt
- 7: Wirkstoffpflaster
- 8: Deckmembran
- 9: Wirkmembran
- 10: Matrix
- 11: Molekularsieb/Wirkstoff-Addukt mit dem Wirkstoff Z₃
- 12: Molekularsieb/Wirkstoff-Addukt mit dem Wirkstoff Z₄
- 13: Hautkontakt

## Patentansprüche

1. Wirkstoffpflaster (1, 7), umfassend wenigstens
- eine Deckmembran (2, 8) und
- eine Wirkmembran (3, 9) auf der Basis einer hautverträglichen Matrix (4, 10), in die ein Molekularsieb eingemischt ist, das mit wenigstens einem Wirkstoff (Z, Z₁, Z₂, Z₃, Z₄) beladen ist, wobei das Molekularsieb/Wirkstoff-Addukt (5, 11, 12) bei Hautkontakt (6, 13) der Wirkmembran in Verbindung mit dem Transpirationswasser der Haut den Wirkstoff freigibt;
**dadurch gekennzeichnet, dass**
- das Molekularsieb/Wirkstoff-Addukt (5, 11, 12) zusätzlich Kristallwasser enthält, und zwar derart, dass in Bezug auf eine ausreichende Grundmolmenge (m) an Kristallwasser das Molekularsieb partiell dehydratisiert ist, wobei das Molekularsieb mit der reduzierten Molmenge (m') an Kristallwasser mit dem Wirkstoff (Z, Z₁, Z₂, Z₃, Z₄) beladen ist, so dass bei Hautkontakt (6, 13) der Wirkmembran (3, 9) eine Adsorption von Wasser unter Desorption des Wirkstoffes stattfindet, wobei der Kristallwassergehalt des Molekularsiebes zunimmt.

2. Wirkstoffpflaster nach Anspruch 1, **dadurch gekennzeichnet, dass** das Molekularsieb ein Natrium-Aluminium-Silikat der folgenden Formel ist:
Na₈₆ [(AlO₂)₈₆ · (SiO₂)₁₀₆] · m(m')H₂O

3. Wirkstoffpflaster nach Ansprüch 1 oder 2, **dadurch gekennzeichnet, dass** das Molekularsieb eine Grundmolmenge (m) an Kristallwasser von wenigstens 100, insbesondere wenigstens 200, enthält.

4. Wirkstoffpflaster nach einem der Ansprüche 1 bis 3, **dadurch gekennzeichnet, dass** das partiell dehydratisierte Molekularsieb einen Dehydratisierungsgrad von mindestens 20 %, vorzugsweise von 40 bis 70 %, aufweist.

5. Wirkstoffpflaster nach einem der Ansprüche 1 bis 4, **dadurch gekennzeichnet, dass** der Beladungsgrad des Wirkstoffes (Z, Z₁, Z₂, Z₃, Z₄) im Molekularsieb/ Wirkstoff-Addukt (5, 11, 12) kleiner ist als der Dehydratisierungsgrad des partiell dehydratisierten Molekularsiebes.

6. Wirkstoffpflaster nach Anspruch 5. **dadurch gekennzeichnet, dass** der Beladungsgrad mindestens 50 % des Dehydratisierungsgrades beträgt.

7. Wirkstoffpflaster nach einem der Ansprüche 1 bis 6, **dadurch gekennzeichnet, dass** das Molekularsieb/Wirkstoff-Addukt (5, 11, 12) innerhalb der Matrix (4, 10) der Wirkmembran (3, 9) im wesentlichen gleichmäßig verteilt ist.

8. Wirkstoffpflaster nach einem der Ansprüche 1 bis 7, **dadurch gekennzeichnet, dass** das Molekularsieb/Wirkstoff-Addukt (5, 11, 12) einen Anteil von 2 bis 20 Gew.-%, vorzugsweise 5 bis 10 Gew.-%, aufweist, und zwar bezogen auf die Gesamtmasse der Wirkmembran (3, 9).

9. Wirkstoffpflaster nach einem der Ansprüche 1 bis 8, **dadurch gekennzeichnet, dass** die Matrix (4, 10) der Wirkmembran (3, 9) ein Polymerwerkstoff, vorzugsweise ein Elastomer, ein thermoplastisches Elastomer oder ein thermoplastischer Kunststoff ist.

10. Wirkstoffpflaster nach einem der Ansprüche 1 bis 9, **dadurch gekennzeichnet, dass** die Wirkmembran (3, 9) eine Schichtdicke von 0,5 mm bis 2 mm, vorzugsweise 1 mm, aufweist.

11. Wirkstoffpflaster nach einem der Ansprüche 1 bis 10, **dadurch gekennzeichnet, dass** die Wirkmembran (3, 9) eine größere Schichtdicke aufweist als die Deckmembran (2, 8).

12. Wirkstoffpflaster nach einem der Ansprüche 1 bis 11, **dadurch gekennzeichnet, dass** das Molekularsieb bei einem einheitlichen Dehydratisierungsgrad mit wenigstens einem ersten Wirkstoff (Z₁) und einem zweiten Wirkstoff (Z₂) beladen ist, die sich durch einen unterschiedlichen Beladungsgrad auszeichnen, so dass das Molekularsieb/Wirkstoff-Addukt (5) bei Hautkontakt (6) der Wirkmembran (3) die Wirkstoffe (Z₁, Z₂) sequentiell freigibt.

13. Wirkstoffpflaster nach einem der Ansprüche 1 bis 11, **dadurch gekennzeichnet, dass** in die Matrix (10) wenigstens ein erstes und zweites Molekularsieb eingemischt sind, die typengleich sind, sich jedoch durch einen unterschiedlichen Dehydratisierungsgrad auszeichnen, wobei das erste Molekularsieb mit einem ersten Wirkstoff (Z₃) und das zweite Molekularsieb mit einem zweiten Wirkstoff (Z₄) beladen sind, und zwar bei etwa gleichem Beladungsgrad, so dass das erste Molekularsieb/Wirkstoff-Addukt (11) und das zweite Molekularsieb/Wirkstoff-Addukt (12) bei Hautkontakt (13) der Wirkmembran (9) die Wirkstoffe (Z₃, Z₄) sequentiell freigeben.

14. Wirkstoffpflaster nach einem der Ansprüche 1 bis 13, **dadurch gekennzeichnet, dass** die Wirkmembran (3, 9) selbstklebend ist.

15. Wirkstoffpflaster nach Anspruch 14, **dadurch gekennzeichnet, dass** die Selbstklebefähigkeit der Wirkmembran (3, 9) auf einer selbstklebenden Matrix (4, 10) beruht.

16. Wirkstoffpflaster nach Anspruch 14, **dadurch gekennzeichnet, dass** das Molekularsieb/Wirkstoff-Addukt (5, 11, 12) so präpariert ist, dass es in Verbindung mit einer nichtklebenden Matrix (4, 10) die Selbstklebefähigkeit der Wirkmembran (3, 9) auslöst.

17. Verfahren zur Herstellung eines Wirkstoffpflasters (1, 7) nach einem der Ansprüche 1 bis 16, **gekennzeichnet durch** folgende Verfahrensschritte:
- das Molekularsieb mit einer ausreichenden Grundmolmenge (m) an Kristallwasser wird bei 300 bis 500°C, vorzugsweise bei 400 bis 450°C, mehrere Stunden, vorzugsweise 2 bis 6 Stunden, lang partiell dehydratisiert;
- anschließend wird das partiell dehydratisierte Molekularsieb mit der reduzierten Molmenge (m') an Kristallwasser mit wenigstens einem Wirkstoff (Z, Z₁, Z₂, Z₃, Z₄) unter Bildung des Molekularsieb/UVirkstoff-Adduktes (5, 11, 12) beladen;
- nun wird das Molekularsieb/Wirkstoff-Addukt (5, 11, 12) in die Matrix (4, 10) unter Bildung der Wirkmembran (3, 9) eingemischt;
- schließlich erfolgt die Konfektionierung der Wirkmembran (3, 9) mit der Deckmembran (2, 8) unter Bildung des Wirkstoffpflasters (1, 7).

18. Verfahren nach Anspruch 17, **dadurch gekennzeichnet, dass** die Dehydratisierung und/oder Beladung in Gegenwart eines inerten Gases, beispielsweise von Stickstoff, durchgeführt wird/werden.

19. Verfahren nach Anspruch 17 oder 18, **dadurch gekennzeichnet, dass** die Dehydratisierung und/oder Beladung unter Normaldruck durchgeführt wird/werden.

20. Verfahren nach einem der Ansprüche 17 bis 19 in Verbindung mit Anspruch 15, **dadurch gekennzeichnet, dass** sich ein Trockenverfahren anschließt.

21. Verfahren nach Anspruch 20, **dadurch gekennzeichnet, dass** das Trockenverfahren bei 30 bis 100°C, vorzugsweise bei 50 bis 70°C, 2 bis 6 Stunden, vorzugsweise 3 bis 4 Stunden, lang durchgeführt wird.

## Claims

1. Plaster containing an active substance (1, 7), comprising at least
- a covering membrane (2, 8) and
- an active membrane (3, 9) based on a skin-compatible matrix (4, 10) into which is mixed a molecular sieve which is charged with at least one active substance (Z, Z₁, Z₂, Z₃, Z₄), the molecular sieve/active substance adduct (5, 11, 12) releasing the active substance when the active membrane comes into contact with the skin (6, 13) in conjunction with the perspiration from the skin,
**characterised in that**
- the molecular sieve/active substance adduct (5, 11, 12) also contains water of crystallisation such that the molecular sieve is partially dehydrated in relation to a sufficient basic molar amount (m) of water of crystallisation, the molecular sieve with the reduced molar amount (m') of water of crystallisation being charged with the active substance (Z, Z₁, Z₂, Z₃, Z₄) so that when the active membrane (3, 9) comes into contact with the skin (6, 13) water is adsorbed and the active substance is desorbed, increasing the level of water of crystallisation contained in the molecular sieve.

2. Plaster containing an active substance according to claim 1, **characterised in that** the molecular sieve is a sodium-aluminium silicate with the following formula:
*Na*₈₆[(*AlO*₂)₈₆ · (*SiO*₂)₁₀₆] · *m*(*m'*)*H₂O*

3. Plaster containing an active substance according to claim 1 or 2, **characterised in that** the molecular sieve contains a basic molar amount (m) of water of crystallisation of at least 100, in particular at least 200.

4. Plaster containing an active substance according to one of claims 1 to 3, **characterised in that** the partially dehydrated molecular sieve exhibits a level of dehydration of at least 20%, preferably 40 to 70%.

5. Plaster containing an active substance according to one of claims 1 to 4, **characterised in that** the level of charging of the active substance (Z, Z₁, Z₂, Z₃, Z₄) in the molecular sieve/active substance adduct (5, 11, 12) is less than the level of dehydration of the partially dehydrated molecular sieve.

6. Plaster containing an active substance according to claim 5, **characterised in that** the level of charging is at least 50% of the level of dehydration.

7. Plaster containing an active substance according to one of claims 1 to 6, **characterised in that** the molecular sieve/active substance adduct (5, 11, 12) is essentially distributed uniformly inside the matrix (4, 10) of the active membrane (3, 9).

8. Plaster containing an active substance according to one of claims 1 to 7, **characterised in that** the molecular sieve/active substance adduct (5, 11, 12) makes up 2 to 20% by weight, preferably 5 to 10% by weight, related to the overall mass of the active membrane (3, 9).

9. Plaster containing an active substance according to one of claims 1 to 8, **characterised in that** the matrix (4, 10) of the active membrane (3, 9) is a polymer material, preferably an elastomer, a thermoplastic elastomer or a thermoplastic plastic.

10. Plaster containing an active substance according to one of claims 1 to 9, **characterised in that** the active membrane (3, 9) exhibits a layer thickness of 0.5 mm to 2 mm, preferably 1 mm.

11. Plaster containing an active substance according to one of claims 1 to 10, **characterised in that** the active membrane (3, 9) exhibits a greater layer thickness than the covering membrane (2, 8).

12. Plaster containing an active substance according to one of claims 1 to 11, **characterised in that** with a uniform level of dehydration the molecular sieve is charged with at least a first active substance (Z₁) and a second active substance (Z₂) which are **characterised by** a different level of charging so that the molecular sieve/active substance adduct (5) releases the active substances (Z₁, Z₂) sequentially when the active membrane (3) comes into contact with the skin (6).

13. Plaster containing an active substance according to one of claims 1 to 11, **characterised in that** at least a first and a second molecular sieve are mixed into the matrix (10), these being of the same type but having a different level of dehydration, the first molecular sieve being charged with a first active substance (Z₃) and the second molecular sieve being charged with a second active substance (Z₄), with roughly the same level of charging, so that the first molecular sieve/active substance adduct (11) and the second molecular sieve/active substance adduct (12) release the active substances (Z₃, Z₄) sequentially when the active membrane (9) comes into contact with the skin (13).

14. Plaster containing an active substance according to one of claims 1 to 13, **characterised in that** the active membrane (3, 9) is self-adhesive.

15. Plaster containing an active substance according to claim 14, **characterised in that** the self-adhesiveness of the active membrane (3, 9) is based on a self-adhesive matrix (4, 10).

16. Plaster containing an active substance according to claim 14, **characterised in that** the molecular sieve/active substance adduct (5, 11, 12) is prepared so that it releases the self-adhesiveness of the active membrane (3, 9) in combination with a non-adhesive matrix (4, 10).

17. Method for manufacturing a plaster containing an active substance (1, 7) according to one of claims 1 to 16, **characterised by** the following process steps:
- the molecular sieve with a sufficient basic molar amount (m) of water of crystallisation is partially dehydrated at 300 to 500°C, preferably at 400 to 450°C, for a plurality of hours, preferably 2 to 6 hours;
- then the partially dehydrated molecular sieve with the reduced molar amount (m') of water of crystallisation is charged with at least one active substance (Z, Z₁, Z₂, Z₃, Z₄) forming the molecular sieve/active substance adduct (5, 11, 12);
- then the molecular sieve/active substance adduct (5, 11, 12) is mixed into the matrix (4, 10) forming the active membrane (3, 9);
- finally the active membrane (3, 9) is produced with the covering membrane (2, 8) forming the plaster containing an active substance (1, 7).

18. Method according to claim 17, **characterised in that** the dehydration and/or charging is/are carried out in the presence of an inert gas, for example nitrogen.

19. Method according to claim 17 or 18, **characterised in that** the dehydration and/or charging is/are carried out under normal pressure.

20. Method according to one of claims 17 to 19 in combination with claim 15, **characterised in that** a drying process follows.

21. Method according to claim 20, **characterised in that** the drying process is carried out at 30 to 100°C, preferably at 50 to 70°C, for 2 to 6 hours, preferably 3 to 4 hours.

## Revendications

1. Emplâtre à principe actif (1, 7), comprenant au moins
- une membrane de recouvrement (2, 8) et
- une membrane active (3, 9) sur la base d'une matrice compatible avec la peau (4, 10), dans laquelle est mélangé un tamis moléculaire qui est chargé avec au moins un principe actif (Z, Z₁, Z₂, Z₃, Z₄), l'adduit tamis moléculaire/principe actif (5, 11, 12) libérant le principe actif lors du contact avec la peau (6, 13) de la membrane active en liaison avec la sueur de la peau,
**caractérisé en ce que**
- 1' adduit tamis moléculaire/principe actif (5, 11, 12) contient en plus de l'eau de cristallisation et ce, de telle manière que, par rapport à une quantité de base suffisante en mole (m) d'eau de cristallisation le tamis moléculaire est partiellement déshydraté, le tamis moléculaire étant chargé de la quantité en mole réduite (m') d'eau de cristallisation avec le principe actif (Z, Z₁, Z₂, Z₃, Z₄), si bien que lors du contact avec la peau (6, 13) de la membrane active (3, 9) une adsorption d'eau a lieu pendant une désorption du principe actif, la teneur en eau de cristallisation du tamis moléculaire augmentant.

2. Emplâtre à principe actif selon la revendication 1, **caractérisé en ce que** le tamis moléculaire est un silicate de sodium et d'aluminium de la formule suivante :
Na₈₆ [(AlO₂)₈₆ . (SiO₂)₁₀₆] . m (m')H₂O

3. Emplâtre à principe actif selon la revendication 1 ou 2, **caractérisé en ce que** le tamis moléculaire contient une quantité de base en mole (m) d'eau de cristallisation d'au moins 100, en particulier d'au moins 200.

4. Emplâtre à principe actif selon l'une des revendications 1 à 3, **caractérisé en ce que** le tamis moléculaire partiellement déshydraté affiche un degré de déshydratation d'au moins 20 %, de préférence de 40 à 70 %.

5. Emplâtre à principe actif selon l'une des revendications 1 à 4, **caractérisé en ce que** le degré de charge du principe actif (Z, Z₁, Z₂, Z₃, Z₄) dans 1' adduit tamis moléculaire/principe actif (5, 11, 12) est inférieur au degré de déshydratation du tamis moléculaire partiellement déshydraté.

6. Emplâtre à principe actif selon la revendication 5, **caractérisé en ce que** le degré de charge correspond à au moins 50 % du degré de déshydratation.

7. Emplâtre à principe actif selon l'une des revendications 1 à 6, **caractérisé en ce que** l' adduit tamis moléculaire/principe actif (5, 11, 12) est pour l'essentiel réparti de manière homogène à l'intérieur de la matrice (4, 10) de la membrane active (3, 9).

8. Emplâtre à principe actif selon l'une des revendications 1 à 7, **caractérisé en ce que** l'adduit tamis moléculaire/principe actif (5, 11, 12) présente une part de 2 à 20 % en poids, de préférence de 5 à 10 % en poids et ce, par rapport à la masse totale de la membrane active (3, 9).

9. Emplâtre à principe actif selon l'une des revendications 1 à 8, **caractérisé en ce que** la matrice (4, 10) de la membrane active (3, 9) est un matériau polymère, de préférence un élastomère, un élastomère thermoplastique ou une matière synthétique thermoplastique.

10. Emplâtre à principe actif selon l'une des revendications 1 à 9, **caractérisé en ce que** la membrane active (3, 9) a une épaisseur de couche de 0,5 mm à 2 mm, de préférence de 1 mm.

11. Emplâtre à principe actif selon l'une des revendications 1 à 10, **caractérisé en ce que** la membrane active (3, 9) a une épaisseur de couche supérieure à celle de la membrane de recouvrement (2, 8).

12. Emplâtre à principe actif selon l'une des revendications 1 à 11, **caractérisé en ce que** le tamis moléculaire est, pour un degré de déshydratation uniforme, chargé avec au moins un premier principe actif (Z₁) et un deuxième principe actif (Z₂) qui se distinguent par un degré de charge différent, de telle manière que l'adduit tamis moléculaire/principes actifs (5) libère les principes actifs (Z₁, Z₂) de manière séquentielle lors du contact avec la peau (6) de la membrane active (3).

13. Emplâtre à principe actif selon l'une des revendications 1 à 11, **caractérisé en ce qu'**au moins un premier et un deuxième tamis moléculaire qui sont du même type, mais se distinguent cependant par un degré de déshydratation différent, sont mélangés dans la matrice (10), le premier tamis moléculaire étant chargé avec un premier principe actif (Z₃) et le deuxième tamis moléculaire avec un deuxième principe actif (Z₄) et ce, pour un degré de charge presque identique, de telle manière que le premier adduit tamis moléculaire/principe actif (11) et le deuxième adduit tamis moléculaire/principe actif (12) libèrent les principes actifs (Z₃, Z₄) de manière séquentielle lors du contact avec la peau (13) de la membrane active (9).

14. Emplâtre à principe actif selon l'une des revendications 1 à 13, **caractérisé en ce que** la membrane active (3, 9) est autocollante.

15. Emplâtre à principe actif selon la revendication 14, **caractérisé en ce que** la capacité de la membrane active (3, 9) à être autocollante repose sur une matrice autocollante (4, 10).

16. Emplâtre à principe actif selon la revendication 14, **caractérisé en ce que** l'adduit tamis moléculaire/principe actif (5, 11, 12) est préparé de telle manière qu'elle déclenche, en liaison avec une matrice non collante (4, 10), la capacité à être autocollante de la membrane active (3, 9).

17. Procédé de fabrication d'un emplâtre à principe actif (1, 7) selon l'une des revendications 1 à 16, **caractérisé par** les étapes de procédé suivantes :
- le tamis moléculaire avec une quantité de base suffisante en mole (m) d'eau de cristallisation est partiellement déshydraté à 300 à 500 °C, de préférence à 400 à 450 °C, pendant plusieurs heures, de préférence pendant 2 à 6 heures ;
- ensuite le tamis moléculaire partiellement déshydraté est chargé avec la quantité réduite en mole (m') d'eau de cristallisation, avec au moins un principe actif (Z, Z₁, Z₂, Z₃, Z₄) pendant la formation de l'adduit tamis moléculaire/principe actif (5, 11, 12) ;
- l'adduit tamis moléculaire/principe actif (5, 11, 12) est maintenant mélangé à la matrice (4, 10) pendant la formation de la membrane active (3, 9) ;
- pour finir, la membrane active (3, 9) est confectionnée avec la membrane de recouvrement (2, 8) lors de la formation de l'emplâtre à principe actif (1, 7).

18. Procédé selon la revendication 17, **caractérisé en ce que** la déshydratation et/ou la charge sont effectuées en présence d'un gaz inerte, par exemple d'azote.

19. Procédé selon la revendication 17 ou 18, **caractérisé en ce que** la déshydratation et/ou la charge sont effectuées à une pression normale.

20. Procédé selon l'une des revendications 17 à 19 en liaison avec la revendication 15, **caractérisé en ce qu'**il est suivi d'un procédé de séchage.

21. Procédé selon la revendication 20, **caractérisé en ce que** le procédé de séchage est effectué à 30 à 100 °C, de préférence à 50 à 70 °C, pendant 2 à 6 heures, de préférence pendant 3 à 4 heures.
